# EUROPÄISCHE PATENTSCHRIFT

(11) **EP 0 783 443 B1**
(45) Veröffentlichungstag und Bekanntmachung des Hinweises auf die Patenterteilung: **15.09.1999**
(21) Anmeldenummer: 95936451.4
(22) Anmeldetag: 06.10.1995
(51) Int. Cl.: B65D 83/16, B65D 83/38

(54) **VORRICHTUNG ZUM BETÄTIGEN VON AEROSOLABGABEGERÄTEN**
AEROSOL DISPENSER ACTUATOR
DISPOSITIF D'ACTIONNEMENT DE DISTRIBUTEURS D'AEROSOLS

(30) Priorität: 10.10.1994 DE 4436051
(43) Veröffentlichungstag der Anmeldung: 16.07.1997
(73) Patentinhaber: Boehringer Ingelheim Pharma KG, 55216 Ingelheim am Rhein (DE)
(72) Erfinder: EICHLER, Gerd, D-55444 Seibersbach (DE); HOCHRAINER, Dieter, D-55411 Bingen am Rhein (DE); REEG, Reiner, D-75173 Pforzheim (DE)
(74) Vertreter: Laudien, Dieter, Dr.
(86) Internationale Anmeldenummer: EP9503951
(87) Internationale Veröffentlichungsnummer: WO9611152

(56) Entgegenhaltungen:
- FR-A- 2 671 294
- GB-A- 1 097 254

## Beschreibung

Die Erfindung betrifft eine Vorrichtung, mit der die Abgabe einer Aerosoldosis aus üblichen Aerosolbehältern erleichtert wird.

Die gebräuchlichen Behälter mit pharmazeutischen Zubereitungen, aus denen bei der Benutzung ein Aerosol abgegeben wird, werden im allgemeinen als "Aerosolbehälter" bezeichnet. Solche Behälter sind indessen mit einer unter einem Druck von mehreren bar stehenden flüssigen Zubereitung gefüllt, von der beim Betätigen eines Ventils eine bestimmte Menge als Sprühstrahl freigesetzt wird und erst dabei mit der Luft das Aerosol bildet. Die Zubereitungen selbst bestehen aus einem verflüssigten Treibgas oder Treibgasgemisch, in dem mindestens ein Wirkstoff und ggf. Hilfsstoffe gelöst oder suspendiert sind.

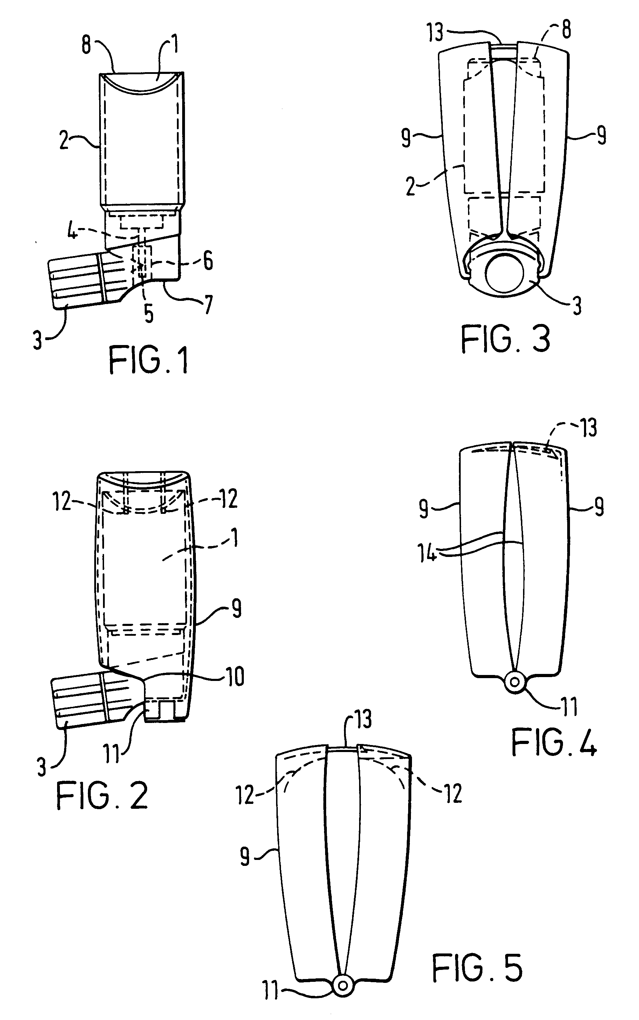

Übliche Aerosolbehälter sind mit Dosierventilen versehen, die bei jeder Betätigung eine festgelegte Menge des Präparats abgeben. Die Freisetzung der Dosis erfolgt, wenn der axial bewegliche Ventilstamm des Dosierventils eingedrückt wird. Der Ventilstamm ist bei den üblichen Geräten in eine entsprechende Aufnahmeeinrichtung eines Mundrohrs gesteckt, das gleichzeitig eine Auflagefläche für den Daumen oder Finger bietet, mit dem der Ventilstamm niedergedrückt wird. Ein typischer Aerosolbehälter dieser Art ist in Fig. 1 dargestellt.

Der Aerosolbehälter 1 befindet sich mit seitlichem bzw. zylindrischem Zwischenraum in einer oben offenen Hülse 2, die mit dem Mundrohr 3 fest verbunden ist. Der Ventilstamm 4 mit der seitlichen Auslaßöffnung 5 ist in der Aussparung 6 des Mundrohrs fixiert. Der Ventilstamm wird durch den Innendruck des Behälters sowie eine Feder nach außen gedrückt; die auf ihn wirkenden Kräfte müssen überwunden werden, um einen Aerosolstoß auszulösen.

Zur Betätigung drückt der Patient meist mit dem Daumen und einem Finger auf die Grifffläche 7 des Mundrohrs und auf den Boden 8 des Aerosolbehälters. Die Auslösung des Aerosolstoßes muß vom Patienten mit seiner Atmung derart koordiniert werden, daß die Aerosolbeimischung zum richtigen Zeitpunkt beim Einatmen erfolgt. Wie sich gezeigt hat, haben viele Patienten mit dieser Koordinierung Schwierigkeiten, etwa wenn bei einem Asthma-Anfall die Applikation besonders rasch erfolgen soll. Dabei kann es vorkommen, daß der Patient sich eine zu geringe Wirkstoffdosis verabreicht. Hat er selbst das Empfinden, keine ausreichende Wirkstoffmenge erhalten zu haben, wird er sich möglicherweise eine weitere Dosis zuführen, was eine Überdosierung ergeben kann. Aus dem britischen Patent 1 097 254 ist eine Auslösehilfe für Dosieraerosole beschrieben, die jedoch nicht für Aerosolbehälter mit einem aufgesteckten Mundrohr geeignet ist. Die Vorrichtung gemäß der im folgenden beschriebenen Erfindung ist ein Zusatz zu den üblichen Aerosolgeräten; sie löst die Aufgabe, das Auslösen des Aerosolstrahls zu erleichtern, wodurch auch die Koordinationsprobleme vermindert werden.

Die erfindungsgemäße Vorrichtung besteht im wesentlichen aus zwei länglichen, mit ihren offenen Seiten einander zugekehrten schalenförmigen Elementen, die gemeinsam ein übliches Aerosolgerät mit Mundrohr zwischen sich aufnehmen können und die an einer Schmalseite durch ein Scharnier schwenkbar verbunden sind, wobei das Mundrohr durch eine Aussparung in der Nähe des Scharniers, im wesentlichen senkrecht zu der Ebene, in der die Schwenkbewegung erfolgt, herausragt, und wobei an der dem Scharnier gegenüberliegenden Schmalseite der schalenförmigen Elemente Einrichtungen vorgesehen sind, die bei der Betätigung der Vorrichtung auf den Boden des Aerosolbehälters drücken (dadurch einen Aerosolstoß auslösend), und beim Loslassen unter dem Druck des Ventilstamms ein Auseinanderschwenken der schalenförmigen Elemente in dem Maß bewirken, daß der Ventilstamm wieder seine Anfangsposition erreicht. Die neue Vorrichtung wird mit der ganzen Hand umfaßt und läßt sich daher besonders leicht auslösen.

Mit den Figuren 2 bis 6 wird die Erfindung anhand einer typischen Ausführungsform näher erläutert.
Fig. 2 zeigt die erfindungsgemäße Vorrichtung von der Seite und läßt in ihrem Inneren das Aerosolgerät mit dem Aerosolbehälter 1, der Hülse 2 und dem Mundrohr 3 erkennen. Das in Betätigungsposition rechte schalenförmige Element 9 ist dem Betrachter zugekehrt. Unterhalb der Aussparung 10 für das Mundrohr 3 ist das Scharnier 11 zu erkennen. Die Einrichtungen 12 sind so geformt, daß sie den Boden 8 des Aerosolbehälters zunehmend nach unten schieben, während die schalenförmigen Elemente 9 zusammengedrückt werden. Werden die schalenförmigen Elemente 9 danach losgelassen, führen die auf den Ventilstamm wirkenden Kräfte dazu, daß der Ventilstamm wieder in seine Ausgangsposition herausgeschoben und damit der Boden 8 nach oben gedrückt wird. Dadurch werden die schalenförmigen Elemente 9 nach außen geschwenkt, so daß das Gerät erneut ausgelöst werden kann.

Fig. 3 zeigt die Ausführungsform nach Fig. 2 in Benutzungshaltung. Die schalenförmigen Elemente 9 befinden sich in der Ausgangsposition. Eine Zunge 13 sorgt dafür, daß die beweglichen Elemente 9 nicht weiter nach außen schwenken können, wenn die Ausgangsposition erreicht ist.

Fig. 4 zeigt das Gerät von der Rückseite in der Endposition bei der Betätigung. Die beiden schalenförmigen Elemente 9 berühren sich an der dem Scharnier 11 gegenüberliegenden Seite. Zwischen den beiden Elementen 9 ist eine durch flache Bogenabschnitte begrenzte Aussparung 14. Durch sie wird vermieden, daß beim Betätigen die Haut der Hand des Patienten eingeklemmt wird. Die Aussparung kann auch eine andere Form haben und eine Entsprechung auf der Vorderseite haben.

Fig. 5 zeigt ebenfalls den Blick auf die Rückseite des Geräts, jedoch in der Ausgangsstellung vor dem Betätigen. Die auf den Boden 8 des Aerosolbehälters drückenden Einrichtungen 12 sind bogenförmig gestaltet, so daß sie beim Zusammendrücken der beiden schalenförmigen Elemente 9 über den Rand des Bodens 8 gleiten und diesen zunehmend weiter nach unten drücken, wodurch der Ventilstamm bis zu seiner Endposition in den Aerosolbehälter hineingeschoben wird.

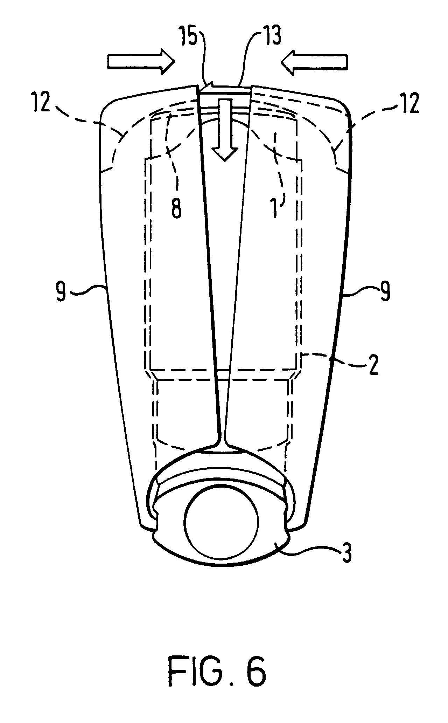

Die Figur 6 zeigt einen Blick auf die Vorderseite des Gerätes mit Frontansicht auf das Mundstück 3 in der Ausgangsstellung vor dem Betätigen. Die Zunge 13 mit dem Widerhaken 15 verhindert, daß die beiden schalenförmigen Elemente 9 nicht unbeabsichtigt auseinanderklappen. In der Abb. ist die Zunge 13 in geöffneter Position, d.h. vor dem Einrasten in das gegenüberliegende Schalenteil 9 dargestellt. In der Abbildung ist das Zusammenwirken der Einrichtungen 12 auf den Boden 8 des Aerosolbehälters 1 beim Zusammendrücken der beiden schalenförmigen Elemente 9 dargestellt.

Die seitliche Anordnung der beiden schalenförmigen Elemente 9 hat sich für die Handhabung als besonders günstig erwiesen, weil eine natürliche und unverkrampfte Haltung der Hand möglich ist.

Die Formen der einzelnen Elemente können vielfältig modifiziert werden. Beispielsweise lassen sich die schalenförmigen Elemente 9 mit überlappenden Teilen versehen, so daß auch in der Ausgangsposition (d. h. wenn die Elemente 9 auseinandergeschwenkt sind) kein Zwischenraum geöffnet ist. Andererseits können die schalenförmigen Elemente 9 durch schmalere Elemente ersetzt werden, wenn ausreichende Steifigkeit gewährleistet ist, und/oder mit Griffmulden versehen sein. Dem Fachmann stehen ferner verschiedene Möglichkeiten zur Verfügung, um die Vorrichtung in der Ausgangsposition zu verriegeln, so daß die Vorrichtung nicht ungewollt betätigt werden kann.

Auch für die Einrichtungen 12 kommen andere Formen in Frage. In den Figuren haben sie die Gestalt von schmalen Rippen mit einer bogenförmigen Begrenzungsfläche, die an der Kante des Aerosolbehälter-Bodens kraftschlüssig vorbeigleitet. Statt bogenförmig kann die Gleitfläche auch in anderer geeigneter Weise gekrümmt oder z. B. keilförmig gestaltet sein. Ferner kann das Scharnier durch ein Verbindungselement aus biegsamem Material ersetzt sein. Um den Betätigungsvorgang deutlicher zu machen, kann die Vorrichtung auch so ausgelegt werden, daß vor der Auslösung des Aerosolstoßes ein Druckpunkt überwunden werden muß.
Schließlich ist es auch möglich, die Vorrichtung asymmetrisch so zu gestalten, daß das Aerosolabgabegerät in dem einen schalenförmigen Element 9 fest angeordnet und nur das andere schalenförmige Element 9 mit einer Einrichtung 12 zum Niederdrücken des Aerosolbehälters ausgestattet ist.

Die erfindungsgemäße Vorrichtung wird vorzugsweise aus einem ausreichend bruchfesten Kunststoff gefertigt.

## Patentansprüche

1. Vorrichtung zum Betätigen von Aerosolabgabegeräten, bestehend aus einem Aerosolbehälter (1) mit einem an einem Ende angeordneten Boden (8) und an einer gegenüberliegenden Ende angeordneten betätigbaren Dosierventil (4,5), das mit einem Mundrohr (3) versehen ist, wobei die Vorrichtung zwei längliche schalenförmige, mit ihren offenen Seiten einander zugekehrte Elemente (9) aufweist, die an einer Schmalseite durch ein Scharnier (11) schwenkbar miteinander verbunden sind und die ein Aerosolabgabegerät zwischen sich aufnehmen können, wobei Einrichtungen (12) an den dem Scharnier (11) gegenüberliegenden Schmalseiten der Elemente (9) vorgesehen sind, die so gestaltet sind, daß sie bei der Betätigung der Vorrichtung durch Drücken auf die Außenseiten der Elemente (9) kraftschlüssig über die Kante des Bodens (8) gleiten, diesen dadurch zunehmend in Richtung des Dosierventils, das dabei betätigt wird, drücken, dadurch gekennzeichnet, daß zwischen den Schmalseiten mit dem Scharnier (11) und den gegenüberliegenden Schmalseiten, unmittelbar neben dem Scharnier (11), eine Aussparung (10) für das Mundrohr (3) des Aerosolabgabegeräts angeordnet ist.

2. Vorrichtung nach Anspruch 1, dadurch gekennzeichnet, daß die Elemente (9) links und rechts von der Symmetrieebene des Aerosolabgabegeräts angeordnet sind.

3. Vorrichtung nach Anspruch 1 und 2, dadurch gekennzeichnet, daß eine Einrichtung vorgesehen ist, welche die unbeabsichtigte Auslösung eines Aerosolstoßes verhindert.

4. Vorrichtung nach Anspruch 1, 2 oder 3, dadurch gekennzeichnet, daß zwischen den Elementen (9) auf der Rückseite (in Benutzungshaltung) und gewünschtenfalls auch auf der Vorderseite eine Aussparung (14) vorgesehen ist.

## Claims

1. A device for actuating aerosol dispensers, comprising an aerosol container (1) having a base (8) arranged at one end and an actuable metering valve (4,5) arranged at the opposite end and provided with a nozzle (3), the device having two elongate, shell-like members (9) facing each other by means of their open sides, which, on one narrow side, are pivotally connected to each other by means of a hinge (11) and are able to receive between them an aerosol dispenser, and means (12) being provided on the narrow sides of the members (9) positioned opposite the hinge (11), which means are constructed in such a way that upon actuation of the device through pressure on the outer sides of the members (9) the means frictionally slide over the edge of the base (8), thereby increasingly depressing the base in the direction of the metering valve which results in the actuation of the valve, characterised in that an opening (10) for the nozzle (3) of the aerosol dispenser is arranged between the narrow sides comprising the hinge (11) and the opposite narrow sides, directly next to the hinge (11).

2. A device according to claim 1, characterised in that the members (9) are arranged to the left and right of the plane of symmetry of the aerosol dispenser.

3. A device according to claim 1 and 2, characterised in that a device is provided which prevents unintentional release of an aerosol spray.

4. A device according to claims 1, 2 or 3, characterised in that a recess (14) is provided between the members (9), on the back thereof (in the service position) and, optionally, also on the front thereof.

## Revendications

1. Dispositif pour actionner des appareils distributeurs d'aérosol, consistant en un récipient à aérosol (1) avec un fond (8) disposé à une extrémité et une valve doseuse (4,5) actionnable disposée à une extrémité opposée, qui est munie d'un tube buccal (3), où le dispositif comporte deux éléments allongés (9) en forme de coquille, tournés l'un vers l'autre par leurs côtés ouverts, qui sont reliés entre eux de manière pivotante par une charnière (11) au niveau d'un petit côté et qui peuvent recevoir entre eux un appareil distributeur d'aérosol, où il est prévu au niveau des petits côtés des éléments (9) opposés à la charnière (11) des organes (12) qui sont agencés de telle manière que, lors de l'actionnement du dispositif par une pression sur les côtés extérieurs des éléments (9), ils glissent par assemblage par force sur l'arête du fond (8), en poussant ainsi celui-ci de manière croissante en direction de la valve doseuse qui est alors actionnée, caractérisé en ce qu'un évidement (10) pour le tube buccal (3) de l'appareil distributeur d'aérosol est agencé entre les petits côtés comportant la charnière (11) et les petits côtés opposés, au voisinage immédiat de la charnière (11).

2. Dispositif selon la revendication 1 caractérisé en ce que les éléments (9) sont disposés à gauche et à droite du plan de symétrie de l'appareil distributeur d'aérosol.

3. Dispositif selon les revendications 1 et 2 caractérisé en ce qu'il est prévu un organe qui empêche le déclenchement involontaire d'une bouffée d'aérosol.

4. Dispositif selon la revendication 1, 2 ou 3 caractérisé en ce qu'un évidement (14) est prévu entre les éléments (9) sur le côté postérieur (en position d'utilisation) et, si on le souhaite, aussi sur le côté antérieur.
